(19) Europäisches Patentamt
European Patent Office
Office européen des brevets

(11) Veröffentlichungsnummer: **0 228 995**
**A1**

(12) # EUROPÄISCHE PATENTANMELDUNG

(21) Anmeldenummer: 86810588.3

(22) Anmeldetag: 15.12.86

(51) Int. Cl.[4]: **C 07 D 249/08**
C 07 D 233/50,
A 01 N 43/653, A 01 N 43/50

(30) Priorität: 20.12.85 CH 5450/85
20.11.86 CH 4656/86

(43) Veröffentlichungstag der Anmeldung:
15.07.87 Patentblatt 87/29

(84) Benannte Vertragsstaaten:
AT BE CH DE ES FR GB GR IT LI LU NL SE

(71) Anmelder: CIBA-GEIGY AG
Klybeckstrasse 141
CH-4002 Basel (CH)

(72) Erfinder: Frick, Willy, Dr.
Alter Kirchweg 15
CH-4148 Pfeffingen (CH)

Meyer, Alfred, Dr.
St. Galler-Ring 220
CH-4054 Basel (CH)

Nyfeler, Robert, Dr.
Bärenfelserstrasse 8
CH-4057 Basel (CH)

(54) **Mikrobizide.**

(57) Neue Verbindungen der Formel

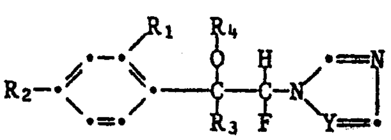

worin
$R_1$ Wasserstoff, Halogen, $C_1$-$C_2$-Alkyl oder $CF_3$;
$R_2$ Halogen oder $C_1$-$C_2$-Alkyl;
$R_3$ $C_1$-$C_5$ Alkyl oder $C_3$-$C_5$-Alkenyl;
$R_4$ Wasserstoff, $C_1$-$C_4$-Alkyl, $C_3$-$C_4$-Alkenyl oder

die Gruppe 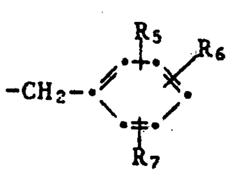 , wobei

$R_5$, $R_6$ und $R_7$ unabhängig voneinander Wasserstoff, Nitro, Halogen, Cyano, $C_1$-$C_3$-Alkyl, $C_1$-$C_3$-Alkoxy, $C_1$-$C_3$-Haloalkoxy oder $CF_3$ darstellt, und
Y ein Stickstoffatom oder die CH-Gruppe bedeuten, einschliesslich ihrer Säureadditionssalze, quaternärer Azoliumsalze und Metallsalzkomplexe mit mikrobiziden Eigenschaften. Die neuen Wirkstoffe können im Pflanzenschutz zur Verhütung des Befalls von Kulturpflanzen durch phytopathogene Mikroorganismen und zur Bekämpfung schädlicher Mirkoorganismen eingesetzt werden.

EP 0 228 995 A1

**Beschreibung**

Mikrobizide

Die vorliegende Erfindung betrifft neue substituierte Aryl-fluorazolyl-alkanole und deren Ether der nachstehenden Formel I sowie deren Säureadditionssalze, quaternäre Azoliumsalze und Metallsalzkomplexe. Die Erfindung betrifft ferner die Herstellung dieser Substanzen sowie mikrobizide Mittel, die als Wirkstoff mindestens eine dieser Verbindungen enthalten. Die Erfindung betrifft ebenso die Herstellung der genannten Mittel sowie die Verwendung der Wirkstoffe oder der Mittel zur Bekämpfung von schädlichen Mikroorganismen, vorzugsweise pflanzenschädigenden Pilzen, insbesondere Piricularia-Arten.

Die erfindungsgemässen Verbindungen entsprechen der allgemeinen Formel I

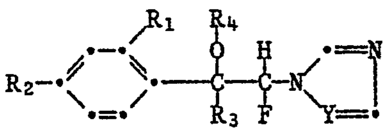

(I),

worin
$R_1$ Wasserstoff, Halogen, $C_1$-$C_2$-Alkyl oder $CF_3$;
$R_2$ Halogen oder $C_1$-$C_2$-Alkyl;
$R_3$ $C_1$-$C_5$-Alkyl oder $C_3$-$C_5$-Alkenyl;
$R_4$ Wasserstoff, $C_1$-$C_4$-Alkyl, $C_3$-$C_4$-Alkenyl oder die Gruppe

die Gruppe 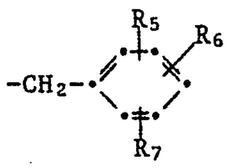 , wobei

wobei
$R_5$, $R_6$ und $R_7$ unabhängig voneinander Wasserstoff, Nitro, Halogen, Cyano, $C_1$-$C_3$-Alkyl, $C_1$-$C_3$-Alkoxy, $C_1$-$C_3$-Haloalkoxy oder $CF_3$ darstellt, und
Y ein Stickstoffatom oder die CH-Gruppe bedeuten,
unter Einschluss ihrer Säureadditionssalze, quaternären Azoliumsalze und Metallsalzkomplexe.

Verbindungen der Formel I, die unter den Umfang der Formel I' fallen,

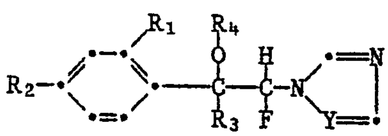

(I'),

worin
$R_1$ Wasserstoff, Halogen, $C_1$-$C_2$-Alkyl oder $CF_3$;
$R_2$ Halogen oder $C_1$-$C_2$-Alkyl;
$R_3$ $C_1$-$C_4$-Alkyl;
$R_4$ Wasserstoff, $C_1$-$C_4$-Alkyl, $C_3$-$C_4$-Alkenyl oder

die Gruppe 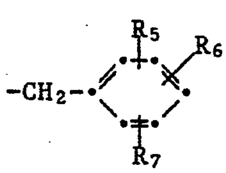 , wobei

$R_5$, $R_6$ und $R_7$ unabhängig voneinander Wasserstoff, Nitro, Halogen, Cyano, $C_1$-$C_3$-Alkyl, $C_1$-$C_3$-Alkoxy, $C_1$-$C_3$-Haloalkoxy oder $CF_3$ darstellt, und
Y ein Stickstoffatom oder die CH-Gruppe bedeuten,
stellen einen engeren Umfang bevorzugter Verbindungen dar.

Unter Alkyl sind je nach Anzahl der angegebenen Kohlenstoffatome beispielsweise Methyl, Ethyl sowie die Isomeren von Propyl und Butyl, wie z.B. n-Propyl, Isopropyl, Isobutyl oder tert.-Butyl zu verstehen. Alkenyl steht z.B. für Propenyl-(1), Allyl, Butenyl-(1), Butenyl-(2) oder Butenyl-(3) und stellt bevorzugt Allyl dar. Halogen bedeutet Fluor, Chlor, Brom oder Jod, vorzugsweise Fluor, Chlor oder Brom.

Die vorliegende Erfindung umfasst sowohl die freien Verbindungen der Formel I als auch deren Säureadditionssalze, quaternäre Azoliumsalze und Metallsalzkomplexe. Die freien Verbindungen im Umfang der Formel I, insbesondere die 1H-1,2,4-Triazolderivate, sind bevorzugt.

Beispiele addierbarer Säuren sind anorganische Säuren: Halogenwasserstoffsäure wie Fluorwasserstoffsäure, Chlorwasserstoffsäure, Bromwasserstoffsäure oder Jodwasserstoffsäure sowie Schwefelsäure, Phosphorsäure, phosphorige Säure, Salpetersäure und organische Säuren wie Essigsäure, Trifluoressigsäure, Trichloressigsäure, Propionsäure, Ameisensäure, Benzolsulfonsäure, p-Toluolsulfonsäure, Methansulfonsäure, Maleinsäure, Bernsteinsäure, Weinsäure, Fumarsäure, Salizylsäure, Milchsäure oder Sorbinsäure.

Die Metallsalzkomplexe bestehen aus der zugrundeliegenden freien Verbindung der Formel I und einem anorganischen oder organischen Metallsalz, beispielsweise den Halogeniden, Nitraten, Sulfaten, Phosphaten, Acetaten, Trifluoracetaten, Trichloracetaten, Propionaten, Tartraten, Sulfonaten, Salicylaten, Benzoaten usw. der Elemente der dritten und vierten Hauptgruppe wie Aluminium, Zinn oder Blei sowie der ersten bis achten Nebengruppe wie Chrom, Mangan, Eisen, Kobalt, Nickel, Zirkonium, Kupfer, Zink, Silver, Quecksilber usw.. Bevorzugt sind die Nebengruppen-Elemente der 4. Periode. Die Metalle können dabei in den verschiedenen ihnen zukommenden Wertigkeiten vorliegen. Die Metallsalzkomplexe der Formel I können ein- oder mehrkernig auftreten, d.h. sie können ein oder mehrere organische Molekülanteile als Liganden enthalten. Komplexe mit den Metallen Kupfer, Zink, Mangan, Zinn und Zirkonium sind bevorzugt.

Zur Herstellung von Quaternierungsprodukten der Verbindungen der Formel (I) kommen vorzugsweise Alkyl- bzw. Aralkylhalogenide, -sulfate oder -sulfonate, wie beispielsweise Methyljodid, Dimethyl-, Diethyl- oder Monomethylsulfat, Benzylchlorid oder -bromid, Methyl-oder Ethyl-p-toluolsulfonat in Frage.

Die Quaternierungsprodukte der Verbindungen der Formel (I) können in einfacher Weise nach üblichen Methoden, wie z.B. durch Umsetzen der Reaktionskomponenten in einem geeigneten Lösungsmittel wie beispielsweise Acetonitril oder Aceton erhalten werden und in bekannter Weise, z.B. durch Ausfällen mit einem geeigneten Fällungsmittel, wie z.B. Diethylether und Absaugen isoliert werden und durch Waschen mit einem inerten organischen Lösungsmittel gereinigt werden.

Die Verbindungen der Formel I sind bei Raumtemperatur stabile Oele, Harze oder überwiegend Feststoffe, die sich durch sehr wertvolle mikrobizide Eigenschaften auszeichnen. Sie lassen sich auf dem Agrarsektor oder verwandten Gebieten präventiv und kurativ zur Bekämpfung von pflanzenschädigenden Mikroorganismen einsetzen, dabei sind die Triazolylmethylderivate im Umfang der Formel I bevorzugt. Die erfindungsgemässen Wirkstoffe der Formel I zeichnen sich bei niedrigen Anwendungskonzentrationen durch eine sehr gute pflanzenfungizide Wirkung und problemlose Anwendung aus.

Aufgrund ihrer ausgeprägten mikrobiziden, insbesondere pflanzenfungiziden Wirkung sind folgende Substanzgruppen bevorzugt:

Verbindungen der Formel I, worin bedeuten:

a) $R_1/R_2$ 2,4-$Cl_2$ oder 2,4-$(CH_3)_2$;
$R_3$ $C_1$-$C_2$-Alkyl;
$R_4$ Wasserstoff, $C_1$-$C_3$-Alkyl, Allyl oder 2,4-Dichlorbenzyl; und
Y ein Stickstoffatom oder die CH-Gruppe.

b) $R_1/R_2$ 2,4-$Cl_2$ oder 2,4-$(CH_3)_2$;
$R_3$ $C_1$-$C_3$-Alkyl oder $C_3$-$C_4$-Alkenyl;
$R_4$ Wasserstoff, $C_1$-$C_3$-Alkyl, Allyl oder 2,4-Dichlorbenzyl; und
Y ein Stickstoffatom oder die CH-Gruppe.

c) $R_1$ Wasserstoff, Chlor oder Methyl;
$R_2$ Fluor, Chlor oder Methyl;
$R_3$ $C_1$-$C_4$-Alkyl oder Allyl;
$R_4$ $C_1$-$C_4$-Alkyl oder $C_3$-$C_4$-Alkenyl; und
Y ein Stickstoff oder die CH-Gruppe.

Davon sind folgende Verbindungen besonders bevorzugt, in welchen darstellen:

unter a) $R_1/R_2$ 2,4-$Cl_2$
$R_3$ $CH_3$
$R_4$ Wasserstoff und
Y ein Stickstoffatom;

unter b) $R_1/R_2$ 2,4-$Cl_2$;
$R_3$ $CH_3$, n-Propyl oder Allyl
$R_4$ Wasserstoff oder Methyl; und
Y ein Stickstoffatom;

unter c) $R_1$ Wasserstoff, Chlor oder Methyl;
$R_2$ Fluor, Chlor oder Methyl;
$R_3$ Methyl, n-Propyl, n-Butyl, tert.Butyl oder Allyl;
$R_4$ Methyl, n-Propyl, n-Butyl, Allyl oder But-2-enyl; und
Y ein Stickstoffatom oder die CH-Gruppe.

In ihrer biologischen Aktivität besonders bevorzugte Verbindungen sind beispielsweise:

1-(2,4-Dichlorphenyl)-1-methyl-2-fluor-2-(1H-1,2,4-triazol-1-yl)-ethanol (Nr. 1.1);
1-(2,4-Dichlorphenyl)-1-n-propyl-2-fluor-2-(1H-1,2,4-triazol-1-yl)-ethanol (Nr. 1.5);
1-(1,2-Dichlorphenyl)-1-n-propyl)-2-fluor-2-(1H-imidazol-1-yl)-ethanol (Nr. 1.6);

1-(2.4-Dimethylphenyl)-1-methyl-2-fluor-2-(1H-1.2,4-triazol-1-yl)-ethanol (Nr. 1.7)
1-(2.4-Dimethylphenyl)-1-n-propyl-2-fluor-2-(1H-1.2.4-triazol-1-yl)-ethanol (Nr. 1.10);
1-(4-fluorphenyl-1-methyl-2-fluor-2-(1H-1.2,4-triazol-1-yl)-ethanol (Nr. 1.11);
1-(4-Chlorphenyl)-1-methyl-2-fluor-2-(1H-1,2,4-triazol-1-yl)-ethanol (Nr. 1.15);
1-(2-Chlor-4-fluorphenyl)-1-n-propyl-2-fluor-2-(1H-1,2.4-triazol-1-yl)-ethanol (Nr. 1.26);
1-(2.4-Dichlorphenyl)-1-n-butyl-2-fluor-2-(1H-1.2.4-triazol-1-yl)-ethanol (Nr. 1.33);
1-(4-Chlorphenyl)-1-t.butyl-2-fluor-2-(1H-1,2.4-triazol-1-yl)-ethanol (Nr. 1.34);
1-(2.4-Dichlorphenyl)-1-allyl-2-fluor-2-(1H-1.2.4-triazol-1-yl)-ethanol (Nr. 1.35);
1-(4-Chlorphenyl)-1-allyl-2-fluor-2-(1H-1.2.4-triazol-1-yl)-ethanol (Nr. 1.36);
1-(2.4-Dichlorphenyl)-1-methyl-1-methoxy-2-fluor-2-(1H-1,2,4-triazol-1-yl)-ethan (Nr. 2.1);
1-(2.4-Dichlorphenyl)-1-methyl-1-n-propoxy-2-fluor-2-(1H-1.2.4-triazol-1-yl)-ethan (Nr. 2.3)
1-(2.4-Dichlorphenyl)-1-methyl-1-n-propoxy-2-(1H-imidazol-1-yl)-ethan (Nr. 2.4)
1-(2.4-Dichlorphenyl)-1-methyl-1-n-butoxy-2-fluor-2-(1H-1,2,4-triazol-1-yl)-ethan (Nr. 2.5);
1-(2.4-Dichlorphenyl)-1-methyl-1-allyloxy-2-fluor-2-(1H-1,2,4-triazol-1-yl)-ethan (Nr. 2.6);
1-(2,4-Dichlorphenyl)-1-methyl-1-allyloxy-2-(1H-imidazol-1-yl)-ethan (Nr. 2.7)
1-(2,4-Dichlorphenyl)-1-methyl-1-but-2-enoxy-2-fluor-2-(1H-1,2,4-triazol-1-yl)-ethan (Nr. 2.8);
1-(2,4-Dichlorphenyl)-1-n-propyl-1-methoxy-2-fluor-2-(1H-1,2,4-triazol-1-yl)-ethan (Nr. 2.15);
1-(2,4-Dichlorphenyl)-1-n-propyl-1-methoxy-2-(1H-imidazol-1-yl)-ethan (Nr. 2.16);
1-(2,4-Dichlorphenyl)-1-n-propyl-1-n-propoxy-2-fluor-2-(1H-1,2,4-triazol-1-yl)-ethan (Nr. 2.17);
1-(2,4-Dichlorphenyl)-1-n-propyl-1-allyloxy-2-fluor-2-(1H-1,2,4-triazol-1-yl)-ethan (Nr. 2.18);
1-(2,4-Dimethylphenyl)-1-methyl-1-methoxy-2-fluor-2-(1H-1,2,4-triazol-1-yl)-ethan (Nr. 2.19);
1-(2,4-Dichlorphenyl)-1-allyl-1-methoxy-2-fluor-2-(1H-1,2,4-triazol-1-yl)-ethan (Nr. 2.51);
1-(2,4-Dichlorphenyl)-1-n-propyl-1-methoxy-2-fluor-2-(1H-1,2,4-triazol-1-yl)-ethan (Nr. 2.52).

Verbindungen der Formel Ia (Alkanolderivate)

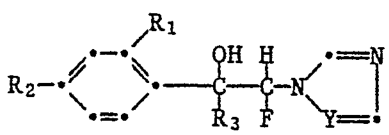 (Ia),

werden erfindungsgemäss hergestellt, indem man ein Ketonderivat der Formel II

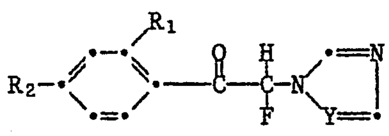 (II),

mit einer Verbindung der Formel IV

$R_3MgX$ (IV)

als Grignard-Reagens in einem inerten Lösungsmittel umsetzt, worin $R_1$, $R_2$ und $R_3$ die unter Formel I angegebenen Bedeutungen haben und X Halogen, insbesondere Brom oder Jod, darstellt.

Die Umsetzung wird nach Art der Grignard-Reaktion (vgl. dazu Nützel, Houben-Weyl Bd. 13/2a, Seiten 47-527) normalerweise in Diethylether durchgeführt; jedoch auch andere Lösungsmittel, z.B. höhere Ether, wie Tetrahydrofuran, Dioxan, Dibutylether oder Anisol sowie ferner Ethylenglykolether oder Diethylenglykolether sind geeignet. Ausserdem können auch Basen wie Pyridin verwendet werden. Als Reaktionstemperaturen kommen solche von -80° bis 50°C, vorzugsweise -60° bis ±0°C in Frage.

Verbindungen der Formel Ib

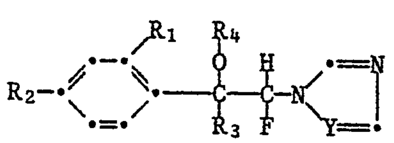 (Ib),

werden erfindungsgemäss hergestellt, indem man eine Verbindung der Formel (Ia)

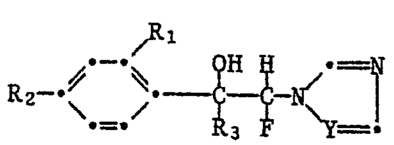 (Ia),

oder eine Verbindung der Formel III

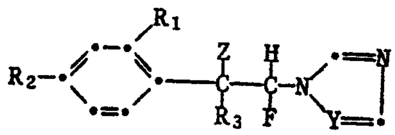

(III),

mit jeweils einer Verbindung der Formel V $R_4-W$ (V) reagieren lässt.

Verbindungen der Formel Ic

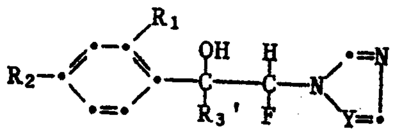

(Ic)

worin $R_3'$ $C_3$-$C_5$-Alkenyl bedeutet, werden erfindungsgemäss hergestellt, indem man eine Verbindung der Formel II

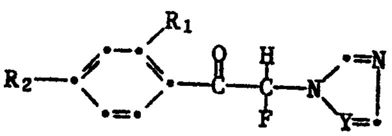

(II)

mit einer Silanverbindung der Formel VI
$R_3'$-Si(Alk)$_3$ (VI)
worin Alk $C_1$-$C_4$-Alkyl, vorzugsweise Methyl, bedeutet, in Gegenwart einer Lewis-Säure umsetzt. (Lit. s. Tetrah. Letters (1976) 1295 - 1298).

Verbindungen der Formel Ic können durch Hydrierung in Gegenwart eines Katalysators, z.B. von Palladium/Aktivkohle, in Verbindungen der Formel Id

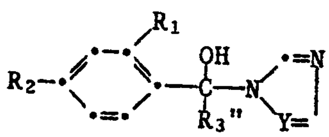

(Id)

worin $R_3''$ $C_3$-$C_5$-Alkyl darstellt, überführt werden.

Darüber hinaus können auch Ether-Verbindungen der Formel Ib, in welchen $R_3$ ein $C_3$-$C_5$-Alkenyl darstellt, in der oben beschriebenen Weise zu den entsprechenden Alkyl-Verbindungen der Formel Ib hydriert werden.

In den vorgängig beschriebenen Verfahren entsprechen $R_1$, $R_2$, $R_3$, $R_4$ und Y den unter Formel I angegebenen Bedeutungen, steht W für -OH, -OM oder eine sogenannte Abgangsgruppe und stellt Z die Gruppe -OM oder eine Abgangsgruppe dar, wobei M ein Alkali- oder Erdalkalimetallatom repräsentiert, mit der Massgabe, dass bei den Reaktanden Ia und III einerseits und V andererseits eine -OM-Gruppe mit einer Abgangsgruppe oder zwei Hydroxy-Gruppen miteinander reagieren.

Unter einer Abgangsgruppe sollen hier und im folgenden Substituenten wie z.B. Halogen (wie Fluor, Chlor, Brom oder Jod, vorzugsweise Chlor oder Brom); Sulfonyloxygruppen, bevorzugt $-OSO_2-R_8$; Acyloxygruppen, bevorzugt $-OCO-R_8$ oder Isoharnstoffreste, bevorzugt

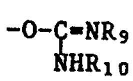

verstanden werden, wobei $R_8$, $R_9$ und $R_{10}$ unabhängig voneinander für $C_1$-$C_3$-Alkyl, $C_1$-$C_3$-Halogenalkyl oder gegebenenfalls durch Halogen, Methyl, Nitro, Trifluormethyl und/oder Methoxy substituiertes Phenyl stehen.

Bei der Reaktion von Verbindungen der Formel Ia mit Formel V, worin W für eine Abgangsgruppe steht, wird die Umsetzung in Abwesenheit oder bevorzugt in Anwesenheit eines reaktionsinerten Lösungsmittels durchgeführt. Dafür eignen sich z.B. folgende Lösungsmittel: N,N-Dimethylformamid, N,N-Dimethylacetamid, Hexamethylphosphortriamid, Dimethylsulfoxid, 2-Methyl-2-pentanon, usw.. Auch Gemische dieser Lösungsmittel untereinander oder mit anderen üblichen inerten organischen Lösungsmitteln, wie z.B. mit aromatischen Kohlenwasserstoffen wie Benzol, Toluol, Xylolen usw. können verwendet werden. Darüber hinaus wird als Lösungsmittel vorzugsweise Wasser verwendet. In manchen Fällen kann es sich als vorteilhaft

erweisen, zur Beschleunigung der Reaktionsgeschwindigkeit in Gegenwart einer Base wie z.B. eines Alkalimetallhydrides, -hydroxides oder -carbonates zu arbeiten. Es kann aber auch von Vorteil sein, dass man das Alkanol der Formel Ia zuerst auf an sich bekannte Weise, z.B. durch Reaktion mit einer starken Base, in ein geeignetes Metallsalz überführt.

Geeignete starke Basen sind z.B. Alkali- und Erdalkalihydride (NaH, KH, $CaH_2$ usw.) und alkaliorganische Verbindungen wie z.B. Butyllithium oder Alkali-tert.-Butoxid. Darüber hinaus ist es auch möglich in wässriger Lösung oder Suspension unter Verwendung wasserlöslicher organischer Lösungsmittel, z.B. Dioxan, Aceton, N,N-Dimethylformamid, als Lösungsvermittler oder in einem zweiphasigen System aus Wasser und einem mit Wasser nicht mischbaren Lösungsmittel, z.B. $CH_2Cl_2$, Ether, Methyl-, Ethylketon, vorzugsweise unter Verwendung von in Wasser löschlichen Basen, wie KOH, NaOH, $Na_2CO_3$, zu arbeiten.

Besonders bei der Verwendung zweiphasiger Systeme ist es von Vorteil, Phasentransferkatalysatoren, z.B. quaternäre Alkylammoniumsalze, zuzusetzen.

In den Fällen, in denen man von einem Alkalialkoholat der Formel III (Z = -OM mit M = Alkalimetall, wie Kalium oder Natrium) ausgeht und dieses mit einer Verbindung der Formel V (worin W für eine Abgangsgruppe steht) umsetzt, arbeitet man vorteilhafterweise in Gegenwart eines Kronenethers. Bei M = K, insbesondere in Gegenwart von 18 Krone-6; bei M = Na, insbesondere in Gegenwart von 15 Krone-5. Dabei wird die Reaktion zweckmässigerweise in einem reaktionsinerten Medium durchgeführt. Als Lösungsmittel eignen sich z.B. Ether und etherartige Verbindungen, z.B. Diniederalkylether (Diethylether, Diisopropylether, tert.-Butylmethylether usw.), Tetrahydrofuran, Dioxan und aromatische Kohlenwasserstoffe wie Benzol, Toluol oder Xylole.

Für die organische, mit Wasser nicht mischbare Phase kommen dabei z.B. folgende Lösungsmittel in Frage: Aliphatische und aromatische Kohlenwasserstoffe, wie Pentan, Hexan, Cyclohexan, Petrolether, Ligroin, Benzol, Toluol, Xylole usw., halogenierte Kohlenwasserstoffe, wie Dichlormethan, Chloroform, Tetrachlorkohlenstoff, Ethylendichlorid, 1,2-Dichlorethan, Tetrachlorethylen usw. oder aliphatische Ether, wie Diethylether, Diisopropylether, t-Butylmethylether usw..

Beispiele geeigneter Phasentransfer-Katalysatoren sind: Tetraalkylammoniumhalogenide, -hydrogensulfate oder -hydroxide wie Tetrabutylammoniumchlorid, -bromid, -jodid; Triethylbenzylammoniumchlorid, -bromid; Tetrapropylammoniumchlorid, -bromid, -jodid; usw.. Als Phasentransfer-Katalysatoren kommen auch Phosphonium-Salze in Betracht. Die Reaktionstemperaturen liegen im allgemeinen zwischen 30° und 130°C, bzw. am Siedepunkt des Lösungsmittels oder Lösungsmittelgemisches.

In den Fällen, in denen Verbindungen der Formel Ia mit solchen der Formel V, worin W eine Hydroxygruppe darstellt, umgesetzt werden, wird vorteilhafterweise eine Kondensationsreaktion durchgeführt. Beide Reaktanten werden in einem geeigneten Lösungsmittel unter Rückfluss erhitzt.

Hierbei können grundsätzlich Lösungsmittel eingesetzt werden, die sich gegenüber den Reaktionspartnern inert verhalten und zweckmässigerweise mit Wasser Azeotrope bilden. Es eignen sich hierzu beispielsweise aromatische Kohlenwasserstoffe, wie Benzol, Toluol, Xylole oder halogenierte Kohlenwasserstoffe, wie Dichlormethan, Chloroform, Tetrachlorkohlenstoff, 1,2-Dichlorethan, Tetrachlorethylen, Chlorbenzol, aber auch etherartige Verbindungen, wie tert.-Butylmethylether, Dioxan und andere. In manchen Fällen kann die Verbindung der Formel III selbst als Lösungsmittel verwendet werden. Bei dieser Kondensationsreaktion arbeitet man zweckmässigerweise in Gegenwart einer starken Säure, z.B. para-Toluolsulfonsäure und bei Siedetemperaturen der azeotropen Mischung.

In anderen Fällen, in denen der Substituent Z in Formel III für eine der üblichen Abgangsgruppen und W in Formel V für eine Hydroxygruppe steht, ist es zweckmässig, das Alkanol der Formel Ia zuerst auf an sich bekannte Weise in ein geeignetes Metallsalz zu überführen und dieses dann mit der Verbindung der Formel III, wie bereits beschrieben, umzusetzen.

Verbindungen der Formel III, worin Z für eine der üblichen, weiter oben definierten Abgangsgruppen steht und die restlichen Substituenten wie unter Formel I definiert sind, können aus den Alkoholen der Formel Ia hergestellt werden, indem man deren freie Hydroxygruppe gegen eine Abgangsgruppe Z austauscht.

Der Austausch der freien Hydroxylgruppe in den Verbindungen der Formel Ia gegen eine Abgangsgruppe Z wird bevorzugt in einem reaktionsinerten Lösungsmittel durchgeführt. Beispiele solcher Lösungsmittel sind: Aromatische und aliphatische Kohlenwasserstoffe wie Benzol, Toluol, Xylole, Petrolether, Ligroin oder Cyclohexan; halogenierte Kohlenwasserstoffe wie Chlorbenzol, Methylenchlorid, Ethylenchlorid, Chloroform, Tetrachlorkohlenstoff oder Tetrachlorethylen; Ether und etherartige Verbindungen wie Diethylether, Diisopropylether, tert.-Butylmethylether, Dimethoxyethan, Dioxan, Tetrahydrofuran oder Anisol; Ester wie Ethylazetat, Propylazetat oder Butylazetat; Nitrile wie Acetonitril oder Verbindungen wie Dimethylsulfoxid, Dimethylformamid und Gemische solcher Lösungsmittel untereinander.

Die Einführung der Abgangsgruppe Z erfolgt nach üblichen Methoden. Bedeutet diese Chlor, so wird als Reagenz z.B. Phosphoroxychlorid, Phosphortrichlorid, Phosphorpentachlorid oder vorzugsweise Thionylchlorid eingesetzt. Man arbeitet im allgemeinen bei Temperaturen von 0° bis +120°C. Im Falle von Z = Brom verwendet man bevorzugt Phosphortribromid oder Phosphorpentabromid und führt die Reaktion bei 0° bis +50°C durch. Steht Z für eine der Gruppen -OCO-$R_8$ oder

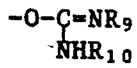

so wird als Reagenz üblicherweise das entsprechende Säurechlorid bzw. Amidinochlorid eingesetzt. Hierbei ist es zweckmässig, wenn die Reaktion bei Temperaturen von -20° bis +50° C, vorzugsweise -10° bis + 30° C, und in Gegenwart einer schwachen Base wie Pyridin oder Triethylamin durchgeführt wird.

Die als Ausgangsstoffe verwendeten Azolylfluorketone der Formel II sind teilsweise aus der Literatur bekannt (vgl. EP-00 27 177). Sie können nach bekannten Methoden hergestellt werden, indem man

a) Verbindungen der Formel VII

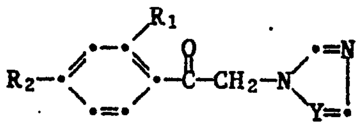

(VII)

mit Brom oder Chlor in Gegenwart eines sauren Lösungsmittels und gegebenenfalls in Gegenwart eines Halogenwasserstoffakzeptors umsetzt und in den entsprechenden Azolyl-brom(chlor)ketonen das Brom(Chlor) in üblicher Weise gegen Fluor austauscht, oder

b) Verbindungen der Formel VIII

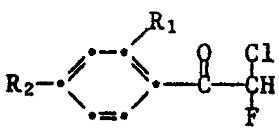

(VIII)

mit Imidazol oder 1,2,4-Triazol in Gegenwart eines Säurebindemittels und in Gegenwart eines Lösungsmittels umsetzt, wobei $R_1$, $R_2$ und Y die unter Formel I angegebenen Bedeutungen haben.

Zur Herstellung der Verbindungen der Formel Ic werden als Lewis-Säuren beispielsweise $TiCl_4$, $AlCl_3$, $GaCl_3$ oder $InCl_3$ verwendet. Die Temperatur beträgt bei dieser Reaktion -10° bis 150° C, vorzugsweise 0° bis 50° C.

Die Hydrierung von Verbindungen der Formeln Ic und Verbindungen der Formel Ib (Ether), in welchen $R_3$ ein $C_3$-$C_5$-Alkenyl darstellt, wird bei Temperaturen von 0° bis 100° C, vorzugsweise 10° bis 50° C, durchgeführt. Als Lösungs- oder Verdünnungsmittel finden hydrierungsinerte Medien Anwendung.

Bei der Herstellung aller hierin genannten Ausgangs-, Zwischen- und Endprodukte können grundsätzlich, sofern nicht ausdrücklich im einzelnen spezifiziert, ein oder mehrere reaktionsinerte Lösungs-oder Verdünnungsmittel anwesend sein. In Frage kommen beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethyl ether usw.) Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander. In manchen Fällen kann es auch von Vorteil sein, wenn die Reaktion oder Teilschritte einer Reaktion unter Schutzgasatmosphäre und/oder absoluten Lösungsmitteln durchgeführt werden und/oder ein Reaktionskatalysator z.B. KJ zugesetzt wird. Als Schutzgase eignen sich inerte Gase wie Stickstoff, Helium, Argon oder in gewissen Fällen auch Kohlendioxid.

Das zur Herstellung der erfindungsgemässen Verbindungen der Formel I beschriebene Verfahren, einschliesslich aller Teilschritte, stellt einen Bestandteil der vorliegenden Erfindung dar.

Die Verbindungen der Formel I besitzen zwei Asymmetriezentren (*)

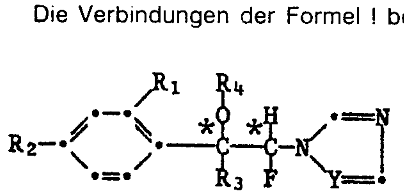

(I),

und bilden daher zwei diastereomere Enantiomerenpaare. Im allgemeinen entsteht bei der Herstellung ein Gemisch der beiden Racemate. das meist durch Kristallisation oder Chromatographie an Silikagel getrennt oder zumindest angereichert werden kann. Wo in den Tabellen 1 und 2 jeweilen zwei Schmelzpunkte angegeben sind, beziehen sich diese auf die beiden Racemate. Die beiden Racemate lassen sich durch die üblichen Methoden der Enantiomerentrennung. z.B. durch fraktionierte Kristallisation der weinsauren Salze oder durch Chromatographie an opt. aktiven Trägern. in die Antipoden zerlegen.

Es wurde nun überraschend gefunden, dass im Gegensatz zu bekannten Fungiziden die erfindungsgemässen Verbindungen der Formel I zusätzlich eine besonders ausgeprägte Aktivität gegen Piricularia-Arten, insbesondere gegen Piricularia oryzae (rice blast) zeigen. Die Pilzspecies Piricularia oryzae gehört zu den Fungi imperfecti und bildet als Fruktifikationsorgane nur Konidiosporen als asexuelle Nebenfruchtform aus. Sie stellt einen der wirtschaftlich wichtigsten Schadfaktoren in Reiskulturen dar, da sie die Reispflanzen in allen Wuchsstadien (Sämlinge, Blätter, Stengel, Aehren usw.) während der ganzen Wachstumsperiode befällt. Je nach Angriffsort des Pilzes und dem Stadium, in dem sich die Reispflanzen bei der Infektion befinden, werden verschiedene englische Bezeichnungen für die Krankheit verwendet: z.B. "Leaf blast" für Blattflecken, die zum Absterben der Blätter und damit zu einem gestörten Wachstum führen können; "Ear blast" für Infektionen an der Rispe oder Teilen der Rispe; "Neckblast" für den Befall der Rispenbasis, wodurch die Rispe abknickt und keine oder nur anormale Körner gebildet werden; "Node blast" für das Abknicken der Halme an den Nodien. Piricularia oryzae verbreitet sich durch Konidiosporen, die bei feuchter Witterung in Massen gebildet werden und vom Wind über weite Strecken transportiert werden. In Gebieten, in denen im Jahresverlauf eine längere Unterbrechung der Reiskultur stattfindet, werden zu Beginn der Anbausaison auf Sämlingen aus infizierten Samen auf Ernterückständen (z.B. auf dem Feld gelagerten Stroh und Gräsern) Konidiosporen gebildet. Wenn das ganze Jahr hindurch Reis angebaut wird, stehen häufig Kulturen verschiedenen Alters nebeneinander, wodurch die Uebertragung der Krankheit durch die sich ständig bildenden Konidien gefördert wird. Der Piriculariabefall hat im allgemeinen sehr hohe Ernteausfälle zur Folge und stellt in Anbetracht der Tatsache, dass Reis nach Weizen und Mais unter den getreideartigen Feldkulturen derzeit weltweit die drittgrösste Anbaufläche beansprucht, einen volkswirtschaftlichen Schaden von grosser Tragweite dar.

Mit den Wirkstoffen der Formel I kann der Piriculariabefall in allen Wuchsstadien erfolgreich und einfach bekämpft werden. Darüber hinaus sind die Wirkstoffe der Formel I auch gegen die den folgenden Klassen angehörenden phytopathogenen Pilze wirksam: Fungi imperfecti (z.B. Botrytis, Helminthosporium, Fusarium, Septoria, Cercospora und Alternaria); Basidiomyceten (z.B. die Gattungen Hemileia, Rhizocotonia, Puccinia); Ascomyceten (z.B. Venturia, Podosphaera, Erysiphe, Monilinia, Uncinula). Ueberdies wirken die Verbindungen der Formel I systemisch. Sie können ferner als Beizmittel zur Behandlung von Saatgut (Früchte, Knollen, Körner) und Pflanzenstecklingen zum Schutz von Pilzinfektionen sowie gegen im Erdboden auftretende phytopathogene Pilze eingesetzt werden.

Ferner lassen sich Verbindungen der Formel I erfolgreich zum Schutz verderblicher Waren pflanzlicher oder tierischer Herkunft einsetzen. Sie bekämpfen z.B. Schimmelpilze wie Penicillium, Aspergillus, Rhizopus, Fusarium, Helminthosporium, Nigrospora und Alternaria sowie Bakterien wie Buttersäurebakterien und Hefen wie Candida.

Die Verbindungen der Formel I können somit im Vorratsschutz (z.B. für Getreide, Silagen, Heu usw.) eingesetzt werden.

Die Verbindungen der Formel I zeigen somit ein für praktische Bedürfnisse sehr günstiges Mikrobizid-Spektrum gegen phytopathogene Pilze und Bakterien. Sie besitzen insbesondere sehr vorteilhafte präventive und zusätzlich systemische Eigenschaften und lassen sich zum Schutz von zahlreichen Kulturpflanzen, vorzugsweise jedoch in Reiskulturen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wodurch auch später zuwachsende Pflanzenteile von derartigen Mikroorganismen verschont bleiben.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. vor allem zur präventiven und systemischen Verhütung eines Befalls an Pflanzen.

Darüber hinaus schliesst die vorliegende Erfindung auch die Herstellung agrochemischer Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Neben der Hauptkultur Reis, gelten als Zielkulturen für die hierin offenbarten Indikationsgebiete im Rahmen dieser Erfindung beispielsweise folgende Pflanzenarten: Getreide (Weizen, Gerste, Roggen, Hafer, Sorghum und Verwandte); Rüben (Zucker- und Futterrüben); Kern-, Stein- und Beerenobst (Aepfel, Birnen, Pflaumen, Pfirsiche, Mandeln, Kirschen, Erd-, Him- und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Pampelmusen, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Compositen).

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen

applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Anzahl der Applikationen richten sich dabei nach dem Befallsdruck für den entsprechenden Erreger (Pilzsorte) oder der Art der Wachstumsbeeinflussung. Die Wirkstoffe der Formel I können aber auch über den Erdboden durch das Wurzelwerk in die Pflanzen gelangen (systemische Wirkung), indem man den Standort der Pflanzen mit einer flüssigen Zubereitung tränkt oder die Substanzen in fester Form in den Boden einbringt, z.B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüber hinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 10 g bis 5 kg Aktivsubstanz (AS) je ha; bevorzugt 100 g bis 2 kg AS/ha, insbesondere bei 200 g bis 600 g AS/ha.

Die Formulierungen d.h. die den Wirkstoff der Formel I und gegebenenfalls eine festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Stoffen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Napththaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht-sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyltaurinsalze zu erwähnen.

Häufiger werden jedoch sog. synthetische Tenside verwendet, insbesondere Fettsulfonate, Fettsulfate, sulfonierte Benzimidazolderivate oder Alkylsulfonate.

Die Fettsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8-22 C-Atomen, Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen 20 bis 250 Aethylenglykoläthergruppen und 10 bis 100 g Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxidaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzoldi(2-chlorethyl)ethylammoniumbromid.

Zur Herstellung von erfindungsgemässen Mitteln, die als Aktivkomponenten die Wirkstoffe der Formel I enthalten, können die vorstehend beschriebenen Tenside zusammen mit weiteren Hilfsstoffen gemäss den in der Formulierungstechnik üblichen Methoden eingesetzt werden.

Die agrochemischen Zubereitungen enthalten in der Regel 0,1 bis 99 %, insbesondere 0,1 bis 95 % Wirkstoff der Formel I, 99,9 bis 1 %, insbesondere 99,8 bis 5 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe einzuschränken.

## Herstellungsbeispiele

### Beispiel 1: Herstellung von 1-(2,4-Dichlorphenyl)-1-methyl-2-fluor-2-(1H-1,2,4-triazol-1-yl)-ethanol

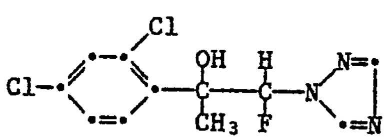

27,4 g 2.4-Dichlor-ω-triazolyl-ω-fluor-acetophenon werden in einem Gemisch von 100 ml Diethylether und 100 ml Tetrahydrofuran gelöst und auf -65°C abgekühlt. Dazu wird unter energischem Rühren eine Grignardlösung, die aus 15 g Methyljodid und 24,5 g Magnesium in 200 ml Diethyläther hergestellt wurde, so zugetropft, dass die Temperatur nicht über -60°C steigt.

Unter Rühren wird das Reaktionsgemisch auf Raumtemperatur erwärmt und bei dieser Temperatur noch eine Stunde belassen. Hierauf werden unter schwachem Kühlen 100 ml einer gesättigten Lösung von Ammoniumchlorid zugetropft.

Zur Aufarbeitung wird die organische Phase abgetrennt, mit Sole neutral gewaschen, über Natriumsulfat getrocknet und unter Vakuum eingedampft. Der Rückstand besteht aus 27,0 g eines hellen Oels.

Bei der Hochdruckchromatographie erweist sich dieses Oel als aus zwei Komponenten bestehend mit einem Peakverhältnis von 1:1,3 (zwei Enantiomerenpaare).

Mit Hilfe von Diisopropylether kann das Oel zur Kristallisation gebracht werden, wobei dasjenige Racemat, das in grösserer Menge vorhanden ist, auskristallisiert und dann bei 149°-155°C schmilzt. Die spektroskopischen Daten stehen mit der erwarteten Struktur in Einklang. Insbesondere ist im IR-Spektrum eine OH-Gruppe deutlich sichtbar. Im Protonen-NMR-Spektrum erscheint die OH-Gruppe bei 6,75 ppm und die $CH_3$-Gruppe ist bei 1,90 ppm sichtbar. Die beiden Peaks der H-F-Kopplung liegen bei 6,98 und 7,61 ppm.

Beispiel 2: Herstellung von
1-Fluor-1-(1-H-1,2,4-triazol-1-yl)-2-methyl-2-methoxy-2-(2,4-dichlorphenyl)-propan

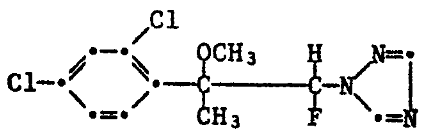

29,0 g der Verbindung von Beispiel 1 werden in 500 ml Methylenchlorid gelöst. Zur Lösung werden bei 0°C 1 g tert.-Butylammoniumbromid und 50 ml 50%-ige Kalilauge zugesetzt. Bei einer Temperatur zwischen 0 und +5°C werden 10 ml Methyljodid zugetropft. Beim Rühren bei 0-5°C fällt im Laufe von zwei Stunden das Kaliumbromid vollständig aus. Zur Vervollständigung der Reaktion rührt man noch zwei Stunden bei Raumtemperatur, worauf man die organische Phase abtrennt, mit Wasser neutral wäscht und mit Natriumsulfat trocknet. Man erhält 30,0 g eines hellen Oels. Dieses kann mit Hilfe von Cyclohexan und Di-iso-propylether zur Kristallisation gebracht werden. Das erhaltene Kristallgemisch schmilzt nach Trocknung zwischen 97° und 103°C.

Die HPLC zeigt, dass es sich praktisch ausschliesslich um ein Gemisch der beiden Enantiomerenpaare handelt.

Das NMR-Spektrum zeigt die beiden $CH_3$-Gruppen bei 2,00 bzw. 3,40 ppm, sowie die beiden Peaks der HF-Kopplung bei 6,60 und 7,35 ppm.

Beispiel 3: Herstellung von 1-(2,4-Dichlorphenyl)-1-allyl-2-fluor-2-(1H-1,2,4-triazol-1-yl)-ethanol

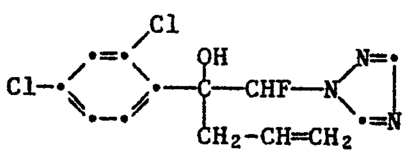

27,4 g 2,4-Dichlor-ω-triazolyl-ω-fluor-acetophenon werden in 150 ml Dichlormethan gelöst. Unter Luftausschluss (im Stickstoffstrom) werden bei 20°C 9,5 g Titantetrachlorid zugesetzt. Nach 5 Minuten Rühren setzt man 11,5 g Trimethyl-allylsilan hinzu, wobei die Temperatur innerhalb von 5 Minuten auf ca. 28°C steigt. Nach 5 Min. wird die braun-rote Emulsion auf Eiswasser gegossen, mit $Na_2CO_3$ vorsichtig alkalisch gestellt und zweimal mit Essigsäureethylester extrahiert.

Die Essigsäureethylesterlösung wird mit Natriumsulfat getrocknet und unter Vakuum eingedampft. Der Rückstand besteht aus 29.0 g eines dunkelbraunen Oels. Beim Abkühlen kann dieses zur Kristallisation gebracht werden. Durch Umkristallisation aus einem Essigsäureethylester/Diethylether-Gemisch erhält man 12,0 g (67 %) eines bei 127° - 128°C schmelzenden Produkts.

Beispiel 4: Herstellung von 2-(2,4-Dichlorphenyl)-1-fluor-1-(1H-1,2,4-triazol-1-yl)-pentanol-(2)

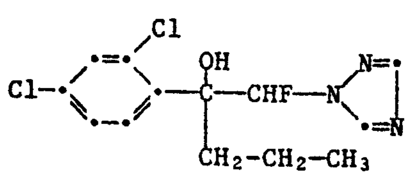

31,6 g der Verbindung von Beispiel 3 werden mit Hilfe von Pd-Aktivkohle in Essigsäureethylester bei Raumtemperatur und unter Normaldruck hydriert. Nach Filtration und Eindampfen unter Vakuum erhält man 31,8 g eines hellen Oels. Mit Hilfe von Essigsäureethylester lässt es sich zur Kristallisation bringen und nach Umkristallisation aus einem Gemisch von Aether und Essigsäureethylester erhält man 26,5 g Kristalle vom Schmelzpunkt 108°-109°C.

Beispiel 5: Herstellung von 1-Fluor-1-(1H-1.2.3-triazol-1-yl)-2-methoxy-2-(2,4-dichlorphenyl)-penten(4)

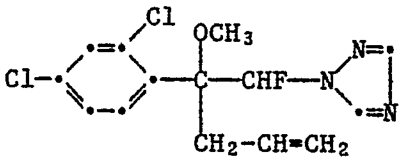

31.6 g der Verbindung von Beispiel 3 werden analog wie im Beispiel 2 beschrieben methyliert. Nach Aufarbeitung erhält man 32.5 g eines hellen Oels. Dieses wird bei 13 Pa einer Kurzwegdestillation bei 140° C unterworfen.

Man erhält 26.2 g farbloses Oel.

Analog den vorstehend beschriebenen Beispielen können die nachfolgend tabellierten Verbindungen hergestellt werden.

Tabelle 1

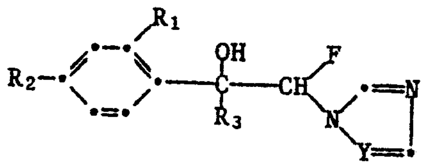

| Nr. | $R_1$ | $R_2$ | $R_3$ | Y | physikalische Daten |
|---|---|---|---|---|---|
| 1.1 | Cl | Cl | $CH_3$ | N | Smp. 149-155°C |
| 1.2 | Cl | Cl | $CH_3$ | CH | |
| 1.3 | Cl | Cl | $C_2H_5$ | N | |
| 1.4 | Cl | Cl | $C_2H_5$ | CH | |
| 1.5 | Cl | Cl | $C_3H_7(n)$ | N | Smp. 108-109°C |
| 1.6 | Cl | Cl | $C_3H_7(n)$ | CH | Oel |
| 1.7 | $CH_3$ | $CH_3$ | $CH_3$ | N | Oel |
| 1.8 | $CH_3$ | $CH_3$ | $C_2H_5$ | N | |
| 1.9 | $CH_3$ | $CH_3$ | $C_2H_5$ | CH | |
| 1.10 | $CH_3$ | $CH_3$ | $C_3H_7(n)$ | N | Oel |
| 1.11 | H | F | $CH_3$ | N | Smp. 130-132°C* / 74-76°C* |
| 1.12 | H | F | $CH_3$ | CH | |
| 1.13 | H | F | $C_2H_5$ | N | |
| 1.14 | H | F | $C_3H_7(n)$ | N | |
| 1.15 | H | Cl | $CH_3$ | N | Smp. 128-130°C* / 86-88°C* |
| 1.16 | H | Cl | $CH_3$ | CH | |
| 1.17 | H | Cl | $C_2H_5$ | N | |
| 1.18 | H | Cl | $C_2H_5$ | CH | |
| 1.19 | H | Cl | $C_3H_7(n)$ | N | |
| 1.20 | H | Br | $CH_3$ | N | |
| 1.21 | H | Br | $C_2H_5$ | N | |
| 1.22 | F | F | $CH_3$ | N | |
| 1.23 | F | F | $C_2H_5$ | N | |
| 1.24 | Cl | F | $CH_3$ | N | |
| 1.25 | Cl | F | $C_2H_5$ | N | |
| 1.26 | Cl | F | $C_3H_7(n)$ | N | Harz |
| 1.27 | Cl | Br | $CH_3$ | N | |

* (= Diastereomerenpaare)

Fortsetzung: Tabelle 1

| Nr. | $R_1$ | $R_2$ | $R_3$ | Y | physikalische Daten |
|---|---|---|---|---|---|
| 1.28 | Cl | Br | $C_2H_5$ | N | |
| 1.29 | Cl | $CH_3$ | $CH_3$ | N | |
| 1.30 | Cl | $CH_3$ | $C_2H_5$ | N | |
| 1.31 | $CH_3$ | H | $CH_3$ | N | Smp. 141-143°C |
| 1.32 | $CH_3$ | Cl | $CH_3$ | N | Smp. 134-135°C |
| 1.33 | Cl | Cl | $C_4H_9$(n) | N | Oel |
| 1.34 | H | Cl | $C_4H_9$(tert) | N | Smp. 128-130°C |
| 1.35 | Cl | Cl | Allyl | N | Smp. 127-128°C |
| 1.36 | H | Cl | Allyl | N | Smp. 106-108°C |

Tabelle 2

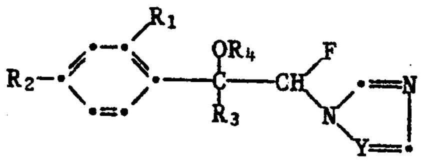

| Nr. | $R_1$ | $R_2$ | $R_3$ | $R_4$ | Y | physikalische Daten |
|---|---|---|---|---|---|---|
| 2.1 | Cl | Cl | $CH_3$ | $CH_3$ | N | Smp. 97-103°C |
| 2.2 | Cl | Cl | $CH_3$ | $CH_3$ | CH | |
| 2.3 | Cl | Cl | $CH_3$ | $C_3H_7(n)$ | N | Oel |
| 2.4 | Cl | Cl | $CH_3$ | $C_3H_7(n)$ | CH | Harz |
| 2.5 | Cl | Cl | $CH_3$ | $C_4H_9(n)$ | N | Oel, $Kp_{1,3p}$ 150°C |
| 2.6 | Cl | Cl | $CH_3$ | $CH_2CH{=}CH_2$ | N | Harz |
| 2.7 | Cl | Cl | $CH_3$ | $CH_2CH{=}CH_2$ | CH | Oel |
| 2.8 | Cl | Cl | $CH_3$ | $CH_2CH{=}CH{-}CH_3$ | N | Harz |
| 2.9 | Cl | Cl | $C_2H_5$ | $CH_3$ | N | |
| 2.10 | Cl | Cl | $C_2H_5$ | $CH_3$ | CH | |
| 2.11 | Cl | Cl | $C_2H_5$ | $C_3H_7(n)$ | N | |
| 2.12 | Cl | Cl | $C_2H_5$ | $CH_2CH(CH_3)_2$ | N | |
| 2.13 | Cl | Cl | $C_2H_5$ | $CH_2CH{=}CH_2$ | N | |
| 2.14 | Cl | Cl | $C_2H_5$ | $CH_2C(CH_3){=}CH_2$ | N | |
| 2.15 | Cl | Cl | $C_3H_7(n)$ | $CH_3$ | N | Oel |
| 2.16 | Cl | Cl | $C_3H_7(n)$ | $CH_3$ | CH | Oel |
| 2.17 | Cl | Cl | $C_3H_7(n)$ | $C_3H_7(n)$ | N | Harz |
| 2.18 | Cl | Cl | $C_3H_7(n)$ | $CH_2CH{=}CH_2$ | N | Oel |
| 2.19 | $CH_3$ | $CH_3$ | $CH_3$ | $CH_3$ | N | Oel |
| 2.20 | $CH_3$ | $CH_3$ | $CH_3$ | $CH_3$ | CH | Oel |
| 2.21 | $CH_3$ | $CH_3$ | $C_2H_5$ | $CH_3$ | N | Oel |
| 2.22 | $CH_3$ | $CH_3$ | $C_3H_7$ | $CH_3$ | N | |
| 2.23 | H | F | $CH_3$ | $CH_3$ | N | |
| 2.24 | H | F | $CH_3$ | $C_4H_9(n)$ | N | |
| 2.25 | H | F | $CH_3$ | $CH_2CH{=}CH{-}CH_3$ | N | |
| 2.26 | H | F | $C_2H_5$ | $CH_3$ | N | |
| 2.27 | H | F | $C_2H_5$ | $C_3H_7(n)$ | N | |
| 2.28 | H | F | $C_2H_5$ | $CH_2{-}CH{=}CH_2$ | N | |
| 2.29 | H | F | $C_3H_7(n)$ | $CH_3$ | N | |

Fortsetzung: Tabelle 2

| Nr. | $R_1$ | $R_2$ | $R_3$ | $R_4$ | Y | physikalische Daten |
|---|---|---|---|---|---|---|
| 2.30 | H | Cl | $CH_3$ | $CH_3$ | N | 115-116°C |
| 2.31 | H | Cl | $CH_3$ | $CH_3$ | CH | |
| 2.32 | H | Cl | $CH_3$ | $C_3H_7(n)$ | N | |
| 2.33 | H | Cl | $CH_3$ | $CH_2CH=CH_2$ | N | |
| 2.34 | H | Cl | $C_2H_5$ | $CH_3$ | N | |
| 2.35 | H | Cl | $C_2H_5$ | $CH_3$ | CH | |
| 2.36 | H | Cl | $C_2H_5$ | $C_3H_7(n)$ | N | |
| 2.37 | H | Cl | $C_2H_5$ | $CH_2CH=CH_2$ | N | |
| 2.38 | H | Cl | $C_3H_7(n)$ | $CH_3$ | N | |
| 2.39 | H | Br | $CH_3$ | $CH_3$ | N | |
| 2.40 | H | Br | $C_2H_5$ | $CH_3$ | N | |
| 2.41 | F | F | $CH_3$ | $CH_3$ | N | |
| 2.42 | F | F | $C_2H_5$ | $CH_3$ | N | |
| 2.43 | Cl | F | $CH_3$ | $CH_3$ | N | |
| 2.44 | Cl | F | $C_2H_5$ | $CH_3$ | N | |
| 2.45 | Cl | F | $C_3H_7$ | $CH_3$ | N | |
| 2.46 | Cl | Br | $CH_3$ | $CH_3$ | N | |
| 2.47 | Cl | Br | $C_2H_5$ | $CH_3$ | N | |
| 2.48 | Cl | $CH_3$ | $CH_3$ | $CH_3$ | N | |
| 2.49 | Cl | $CH_3$ | $C_2H_5$ | $CH_3$ | N | |
| 2.50 | Cl | Cl | $CH_3$ | $CH_2-C_6H_3(Cl)_2(2,4)$ | N | 140-142°C |
| 2.51 | Cl | Cl | Allyl | $CH_3$ | N | Oel |
| 2.52 | Cl | Cl | $C_3H_7(n)$ | $CH_3$ | N | Oel |

Tabelle 3

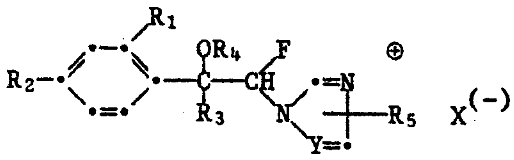

| Nr. | $R_1$ | $R_2$ | $R_3$ | $R_4$ | $R_5$ | Y | X | physikal. Daten |
|---|---|---|---|---|---|---|---|---|
| 3.1 | Cl | Cl | $CH_3$ | $CH_3$ | $CH_3$ | N | $CH_3OSO_3$ | Smp. 164°-166°C |

Formulierungsbeispiele (% = Gewichtsprozent)

| 1. Emulsions-Konzentrate | a) | b) | c) |
|---|---|---|---|
| Wirkstoff aus den Tabellen | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenyl-polyethylenglykolether (30 Mol Ethylenoxid) | - | 12 % | 4 % |
| Cyclohexan | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff aus den Tabellen | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |

(MG = Molekulargewicht)

Die Lösungen sind zur Anwendung in Form kleinster Tropfen geeignet.

| 3. Granulate | a) | b) |
|---|---|---|
| Wirkstoff aus den Tabellen | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst auf den Träger aufgespruht und das Lösungsmittel anschliessend im Vakuum abgedampft.

| 4. Stäubemittel | a) | b) |
|---|---|---|
| Wirkstoff aus den Tabellen | 2 % | 5 % |
| Hochdisperse Kieselsäure | 1 % | 5 % |
| Talkum | 97 % | - |
| Kaolin | - | 90 % |

Durch inniges Vermischen auf Trägerstoffe mit dem Wirkstoff erhält man gebrauchsfertige Stäubemittel.

| 5. Spritzpulver | a) | b) | c) |
|---|---|---|---|
| Wirkstoff aus den Tabellen | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykol ether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

6. Emulsions-Konzentrat

Wirkstoff aus den Tabellen 10 %
Octylphenolpolyethylenglykolether (4-5 Mol Ethylenoxid) 3 %
Ca-Dodecylbenzolsulfonat 3 %
Ricinusölpolyglykolether (35 Mol Ethylenoxid) 4 %
Cyclohexanon 30 %
Xylolgemisch 50 %

Aus diesem Konzentrat können durch Verdünnen mit Wasser Emulsionen jeder gewünschten Konzentration hergestellt werden.

| 7. Stäubemittel | a) | b) |
|---|---|---|
| Wirkstoff aus den Tabellen | 5 % | 8 % |
| Talkum | 95 % | - |
| Kaolin | - | 92 % |

Man erhält anwendungsfertige Stäubemittel, indem der Wirkstoff mit den Träger vermischt auf einer

geeigneten Mühle vermahlen wird.

8. Extruder-Granulat

Wirkstoff aus den Tabellen 10 %
Na-Ligninsulfonat 2 %
Carboxymethylcellulose 1 %
Kaolin 87 %

Der Wirkstoff wird mit den Zusatzstoffen vermischt, vermahlen und mit Wasser angefeuchtet. Dieses Gemisch wird extrudiert und anschliessend im Luftstrom getrocknet.

9. Umhüllungs-Granulat

Wirkstoff aus den Tabellen 3 %
Polyethylenglykol (MG 200) 3 %
Kaolin 94 %
(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

10. Suspensions-Konzentrat

Wirkstoff aus den Tabellen 40 %
Ethylenglykol 10 %
Nonylphenolpolyethylenglykolether (15 Mol Ethylenoxid) 6 %
N-Ligninsulfonat 10 %
Carboxymethylcellulose 1 %
37%ige wässrige Formaldehyd-Lösung 0,2 %
Silikonöl in Form einer 75%igen wässrigen Emulsion 0,8 %
Wasser 32 %

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

Beispiel 1: Wirkung gegen Piricularia oryzae auf Reis

a) Residual-protektive Wirkung

Reispflanzen wurden nach zweiwöchiger Anzucht mit einer aus Spritzpulver des Wirkstoffes hergestellten Spritzbrühe (0,02 % Aktivsubstanz) besprüht. Nach 48 Stunden wurden die behandelten Pflanzen mit einer Konidiensuspension des Pilzes infiziert. Nach 5 Tagen Inkubation bei 95-100 % relativer Luftfeuchtigkeit und 24°C wurde der Pilzbefall beurteilt.

b) Systemische Wirkung

Zu zweiwöchigen Reispflanzen wurde eine aus Spritzpulver des Wirkstoffes hergestellte Spritzbrühe gegossen (0,006 % Aktivsubstanz bezogen auf das Erdvolumen). Darauf wurden die Töpfe mit Wasser soweit gefüllt, dass die untersten Stengelteile der Reispflanzen im Wasser standen. Nach 96 Stunden wurden die behandelten Reispflanzen mit einer Konidiensuspension des Pilzes infiziert. Nach einer Inkubation der infizierten Pflanzen während 5 Tagen bei 95-100 % relativer Luftfeuchtigkeit und ca. 24°C wurde der Pilzbefall beurteilt.

Die unbehandelten aber infizierten Kontrollpflanzen wiesen in den beiden Tests a und b einen Pilzbefall von 100 % auf. Reispflanzen, die mit Verbindungen aus den Tabellen behandelt wurden zeigten eine Reduktion des Pilzbefalls auf 0 bis 25 %. Die Verbindungen Nr. 1.1, 1.5, 1.6, 1.7, 1.10, 1.11, 1.15, 1.26, 1.33, 1.34-1.36, 2.1, 2.3-2.8, 2.15-2.19, 2.51 und 2.52 unterdrückten den Pilzbefall vollständig (0 % Befall).

Beispiel 2: Wirkung gegen Puccinia graminis auf Weizen

a) Residual-protektive Wirkung

Weizenpflanzen wurden 6 Tage nach der Aussaat mit einer aus Spritzpulver des Wirkstoffes hergestellten Spritzbrühe (0.02 % Aktivsubstanz) besprüht. Nach 24 Stunden wurden die behandelten Pflanzen mit einer Uredosporensuspension des Pilzes infiziert. Nach einer Inkubation während 48 Stunden bei 95-100 % relativer Luftfeuchtigkeit und ca. 20 C wurden die infizierten Pflanzen in einem Gewächshaus bei ca. 22°C aufgestellt. Die Beurteilung der Rostpustelentwicklung erfolgte 12 Tage nach der Infektion.

b) Systemische Wirkung

Zu Weizenpflanzen wurden 5 Tage nach Aussaat eine aus Spritzpulver des Wirkstoffes hergestellte Spritzbrühe gegossen (0,006 % Aktivsubstanz bezogen auf das Bodenvolumen). Nach 72 Stunden wurden die

behandelten Pflanzen mit einer Uredosporensuspension des Pilzes infiziert. Nach einer Inkubation während 48 Stunden bei 95-100 % relativer Luftfeuchtigkeit und ca. 20° C wurden die infizierten Pflanzen in einem Gewächshaus bei ca. 22° C aufgestellt. Die Beurteilung der Rostpustelnentwicklung erfolgte 12 Tage nach der Infektion.

Verbindungen aus den Tabellen zeigten gegen Puccinia-Pilze eine sehr gute Wirkung. Unbehandelte aber infizierte Kontrollpflanzen wiesen eine Puccinia-Befall von 100 % auf. Unter anderen hemmten die Verbindungen Nr. 1.1, 1.5, 1.6, 1.7, 1.10, 1.11, 1.15, 1.26, 1.33, 1.34-1.36, 2.1, 2.3-2.8, 2.15-2.19, 2.51 und 2.52 den Puccinia-Befall fast vollständig (0 bis 5 % Befall).

Beispiel 3: Wirkung gegen Erysiphe graminis auf Gerste

a) Residual-protektive Wirkung

Ca. 8 cm hohe Gerstenpflanzen wurden mit einer aus Spritzpulver des Wirkstoffes hergestellten Spritzbrühe (0.02 % Aktivsubstanz) besprüht. Nach 24 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

b) Systemische Wirkung

Zu ca. 8 cm hohen Gerstenpflanzen wurde eine aus Spritzpulver des Wirkstoffes hergestellte Spritzbrühe gegossen (0,006 % Aktivsubstanz bezogen auf das Erdvolumen). Es wurde dabei darauf geachtet, das die Spritzbrühe nicht mit den oberirdischen Pflanzenteilen in Berührung kam. Nach 72 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

Verbindungen der Formel I zeigten gute Wirkung gegen Erysiphe-Pilze. Unbehandelte, aber infizierte Kontrollpflanzen wiesen einen Erysiphe-Befall von 100 % auf. Unter anderen Verbindungen aus den Tabellen hemmten die Verbindungen Nr. 1.1, 1.5, 1.6, 1.7, 1.10, 1.11, 1.15, 1.26, 1.33, 1.34-1.36, 1.34-1.36, 2.1, 2.3-2.8, 2.15-2.19, 2.51 und 2.52 den Pilzbefall bei Gerste auf 0 bis 5 %.

Beispiel 4: Wirkung gegen Cercospora arachidicola auf Erdnusspflanzen

a) Residual-protektive Wirkung

10-15 cm hohe Erdnusspflanzen wurden mit einer aus Spritzpulver der Wirksubstanz hergestellten Spritzbrühe (0,006 % Aktivsubstanz) besprüht und 48 Stunden später mit einer Konidiensuspension des Pilzes infiziert. Die infizierten Pflanzen wurden während 72 Stunden bei ca. 21°C und hoher Luftfeuchtigkeit inkubiert und anschliessend bis zum Auftreten der typischen Blattflecken in einem Gewächshaus aufgestellt.

Die Beurteilung der fungiziden Wirkung erfolgt 12 Tage nach der Infektion basierend auf Anzahl und Grösse der auftretenden Flecken.

Systemische Wirkung

b) Zu 10-15 cm hohen Erdnusspflanzen wurde eine aus Spritzpulver des Wirkstoffes hergestellte Spritzbrühe gegossen (0,06 % Aktivsubstanz bezogen auf das Erdvolumen). Nach 48 Stunden wurden die behandelten Pflanzen mit einer Konidiensuspension des Pilzes infiziert und 72 Stunden bei ca. 21°C und hoher Luftfeuchtigkeit inkubiert. Anschliessend wurden die Pflanzen in, Gewächshaus aufgestellt und nach 11 Tagen der Pilzbefall beurteilt.

Im Vergleich zu unbehandelten, aber infizierten Kontrollpflanzen (Anzahl und Grösse der Flecken = 100 %), zeigten Erdnusspflanzen, die mit Wirkstoffen aus den Tabellen behandelt wurden, einen stark reduzierten Cercospora-Befall. So verhinderten die Verbindungen Nr. 1.1, 1.5, 1.6, 1.7, 1.10, 1.11, 1.15, 1.26, 1.33, 1.34-1.36, 2.1, 2.3-2.8, 2.15-2.19, 2.51 und 2.52 in obigen Versuchen das Auftreten von Flecken fast vollständig (0 bis 10 %).

Beispiel 5: Residual-protektive Wirkung gegen Venturia inaequalis auf Apfeltrieben

Apfelstecklinge mit 10-20 cm langen Frischtrieben wurden mit einer aus Spritzpulver des Wirkstoffes hergestellten Spritzbrühe (0,06 % Aktivsubstanz) besprüht. Nach 24 Stunden wurden die behandelten Pflanzen mit einer Konidiensuspension des Pilzes infiziert. Die Pflanzen inkubiert und während 10 weiteren Tagen in einem Gewächshaus bei 20-24°C aufgestellt. Der Schorfbefall wurde 15 Tage nach der Infektion beurteilt. Verbindungen Nr. 1.1, 1.5, 1.6, 1.7, 1.10, 1.11, 1.15, 1.26, 1.33, 1.34-1.36, 2.1, 2.3-2.8, 2.15-2.19, 2.51 und 2.52 hemmten den Krankheitsbefall auf weniger als 10 %. Kontrollpflanzen waren 100%ig befallen.

**Patentansprüche**

1. Verbindungen der Formel I

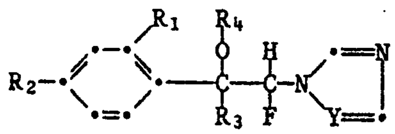

(I),

worin
$R_1$ Wasserstoff, Halogen, $C_1$-$C_2$-Alkyl oder $CF_3$;
$R_2$ Halogen oder $C_1$-$C_2$-Alkyl;
$R_3$ $C_1$-$C_5$-Alkyl oder $C_3$-$C_5$-Alkenyl;
$R_4$ Wasserstoff, $C_1$-$C_4$-Alkyl, $C_3$-$C_4$-Alkenyl oder die Gruppe

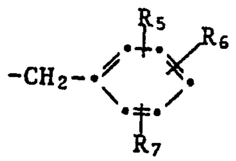

,

wobei
$R_5$, $R_6$ und $R_7$ unabhängig voneinander Wasserstoff, Nitro, Halogen, Cyano, $C_1$-$C_3$-Alkyl, $C_1$-$C_3$-Alkoxy, $C_1$-$C_3$-Haloalkoxy oder $CF_3$ darstellt, und
Y ein Stickstoffatom oder die CH-Gruppe bedeuten,
unter Einschluss ihrer Säureadditionssalze, quaternären Azoliumsalze und Metallsalzkomplexe.

2. Verbindungen der Formel I'

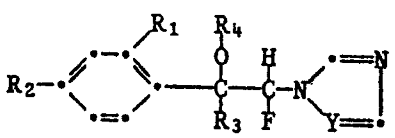

(I'),

worin
$R_1$ Wasserstoff, Halogen, $C_1$-$C_2$-Alkyl oder $CF_3$;
$R_2$ Halogen oder $C_1$-$C_2$-Alkyl;
$R_3$ $C_1$-$C_4$-Alkyl;
$R_4$ Wasserstoff, $C_1$-$C_4$-Alkyl, $C_3$-$C_4$-Alkenyl oder die Gruppe

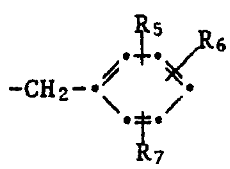

,

wobei
$R_5$, $R_6$ und $R_7$ unabhängig voneinander Wasserstoff, Nitro, Halogen, Cyano, $C_1$-$C_3$-Alkyl, $C_1$-$C_3$-Alkoxy, $C_1$-$C_3$-Haloalkoxy oder $CF_3$ darstellt, und
Y ein Stickstoffatom oder die CH-Gruppe bedeuten.
unter Einschluss ihrer Säureadditionssalze, quaternären Azoliumsalze und Metallsalzkomplexe.

3. Verbindungen der Formel I' nach Anspruch 2, worin
$R_1/R_2$ 2,4-$Cl_2$ oder 2,4-$(CH_3)_2$;
$R_3$ $C_1$-$C_2$-Alkyl; und
$R_4$ Wasserstoff, $C_1$-$C_3$-Alkyl, Allyl oder 2,4-Dichlorbenzyl und
Y ein Stickstoffatom oder die CH-Gruppe bedeuten.

4. Verbindungen der Formel I' nach Anspruch 2, worin
$R_1/R_2$ 2,4-$Cl_2$;
$R_3$ $CH_3$; und
$R_4$ Wasserstoff; und
Y ein Stickstoffatom

bedeuten.

5. Verbindungen der Formel I nach Anspruch 1. worin
$R_1/R_2$ 2.4-$Cl_2$ oder 2,4-$(CH_3)_2$:
$R_3$ $C_1$-$C_3$-Alkyl oder $C_3$-$C_4$-Alkenyl:
$R_4$ Wasserstoff. $C_1$-$C_3$-Alkyl, Allyl oder 2.4-Dichlorbenzyl: und
Y einStickstoffatom oder die CH-Gruppe
bedeuten.

6. Verbindungen der Formel I nach Anspruch 1. worin
$R_1$ Wasserstoff. Chlor oder Methyl:
$R_2$ Fluor. Chlor oder Methyl:
$R_3$ $C_1$-$C_4$-Alkyl oder Allyl:
$R_4$ $C_1$-$C_4$-Alkyl oder $C_3$-$C_4$-Alkenyl: und
Y ein Stickstoff oder die CH-Gruppe
bedeuten.

7. Verbindungen der Formel I nach Anspruch 5, worin
$R_1/R_2$ 2,4-$Cl_2$;
$R_3$ $CH_3$; n-Propyl oder Allyl;
$R_4$ Wasserstoff oder Methyl; und
Y ein Stickstoffatom
bedeuten.

8. Verbindungen der Formel I nach Anspruch 6, worin
$R_1$ Wasserstoff, Chlor oder Methyl;
$R_2$ Fluor, Chlor oder Methyl;
$R_3$ Methyl, n-Propyl, n-Butyl, tert. Butyl oder Allyl;
$R_4$ Methyl, n-Propyl. n-Butyl, Allyl oder But-2-enyl: und
Y ein Stickstoffatom oder die CH-Gruppe
bedeuten.

9. Eine Verbindung der Formel I' nach Anspruch 2 aus der Gruppe:
1-(2,4-Dichlorphenyl)-1-methyl-2-fluor-2-(1H-1,2,4-triazol-1-yl)-ethanol;
1-(2,4-Dichlorphenyl)-1-methyl-1-methoxy-2-fluor-2-(1H-1,2,4-triazol-1-yl)-ethan.

10. Eine Verbindung der Formel I nach Anspruch 1 aus der Gruppe:
1-(2,4-Dichlorphenyl)-1-n-propyl-2-fluor-2-(1H-1,2,4-triazol-1-yl)-ethanol;
1-(1,2-Dichlorphenyl)-1-n-propyl)-2-fluor-2-(1H-imidazol-1-yl)-ethanol;
1-(2,4-Dimethylphenyl)-1-methyl-2-fluor-2-(1H-1.2,4-triazol-1-yl)-ethanol;
1-(2,4-Dimethylphenyl)-1-n-propyl-2-fluor-2-(1H-1,2,4-triazol-1-yl)-ethanol;
1-(4-fluorphenyl-1-methyl-2-fluor-2-(1H-1,2,4-triazol-1-yl)-ethanol;
1-(4-Chlorphenyl)-1-methyl-2-fluor-2-(1H-1,2,4-triazol-1-yl)-ethanol;
1-(2-Chlor-4-fluorphenyl)-1-n-propyl-2-fluor-2-(1H-1,2,4-triazol-1-yl)-ethanol;
1-(2,4-Dichlorphenyl)-1-n-butyl-2-fluor-2-(1H-1,2,4-triazol-1-yl)-ethanol;
1-(4-Chlorphenyl)-1-t.butyl-2-fluor-2-(1H-1,2,4-triazol-1-yl)-ethanol;
1-(2,4-Dichlorphenyl)-1-allyl-2-fluor-2-(1H-1,2,4-triazol-1-yl)-ethanol;
1-(4-Chlorphenyl)-1-allyl-2-fluor-2-(1H-1,2,4-triazol-1-yl)-ethanol;
1-(2,4-Dichlorphenyl)-1-methyl-1-methoxy-2-fluor-2-(1H-1,2,4-triazol-1-yl)-ethan;
1-(2,4-Dichlorphenyl)-1-methyl-1-n-propoxy-2-fluor-2-(1H-1,2,4-triazol-1-yl)-ethan;
1-(2,4-Dichlorphenyl)-1-methyl-1-n-propoxy-2-(1H-imidazol-1-yl)-ethan;
1-(2,4-Dichlorphenyl)-1-methyl-1-n-butoxy-2-fluor-2-(1H-1,2,4-triazol-1-yl)-ethan;
1-(2,4-Dichlorphenyl)-1-methyl-1-allyloxy-2-fluor-2-(1H-1,2,4-triazol-1-yl)-ethan;
1-(2,4-Dichlorphenyl)-1-methyl-1-allyloxy-2-(1H-imidazol-1-yl)-ethan;
1-(2,4-Dichlorphenyl)-1-methyl-1-but-2-enoxy-2-fluor-2-(1H-1,2,4-triazol-1-yl)-ethan;
1-(2,4-Dichlorphenyl)-1-n-propyl-1-methoxy-2-fluor-2-(1H-1,2,4-triazol-1-yl)-ethan;
1-(2,4-Dichlorphenyl)-1-n-propyl-1-methoxy-2-(1H-imidazol-1-yl)-ethan;
1-(2,4-Dichlorphenyl)-1-n-propyl-1-n-propoxy-2-fluor-2-(1H-1,2,4-triazol-1-yl)-ethan;
1-(2,4-Dichlorphenyl)-1-n-propyl-1-allyloxy-2-fluor-2-(1H-1,2,4-triazol-1-yl)-than;
1-(2.4-Dimethylphenyl)-1-methyl-1-methoxy-2-fluor-2-(1H-1,2,4-triazol-1-yl)-ethan;
1-(2,4-Dichlorphenyl)-1-allyl-1-methoxy-2-fluor-2-(1H-1,2,4-triazol-1-yl)-ethan;
1-(2,4-Dichlorphenyl)-1-n-propyl-1-methoxy-2-fluor-2-(1H-1,2,4-triazol-1-yl)-ethan.

11. Verfahren zur Herstellung von Verbindungen der Formel I, dadurch gekennzeichnet, dass
a) ein Alkanolderivat der Formel Ia

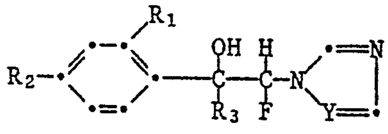

(Ia),

synthetisiert wird, indem man eine Ketonverbindung der Formel II

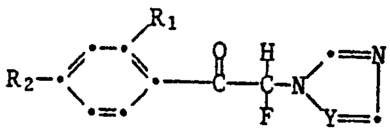

(II),

mit einer Verbindung der Formel IV

$R_3MgX$ (IV)

als Grignard-Reagens in einem inerten Lösungsmittel umsetzt, und

b) ein Etherderivat der Formel Ib

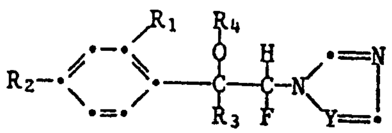

(Ib),

synthetisiert wird, indem man eine Alkanolverbindung der Formel Ia

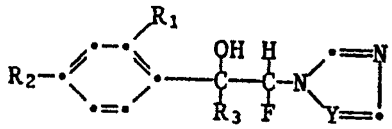

(Ia),

oder eine Verbindung der Formel III

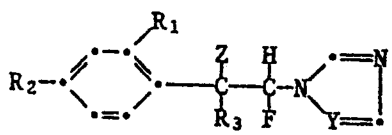

(III),

mit jeweils einer Verbindung der Formel V

$R_4 - W$(V)

reagieren lässt,

und

c) eine Verbindung der Formel Ic

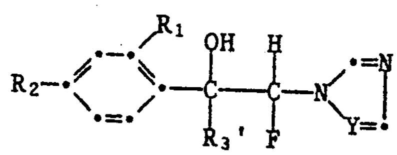

(Ic)

worin $R_3'$ $C_3$-$C_5$-Alkenyl bedeutet, synthetisiert wird, indem man eine Verbindung der Formel II

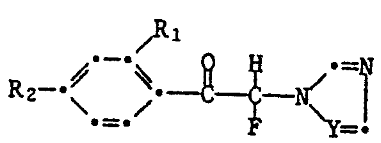

(II)

mit einer Silanverbindung der Formel VI

$R_3'$-$Si(Alk)_3$ (VI)

worin Alk $C_1$-$C_4$-Alkyl, vorzugsweise Methyl, bedeutet, in Gegenwart einer Lewis-Säure umsetzt, und

d) eine Verbindung der Formel Id

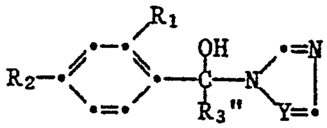

(Id)

worin $R_3''$ $C_3$-$C_5$-Alkyl bedeutet, hergestellt wird, indem man eine Verbindung der Formel Ic

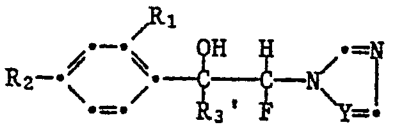

(Ic)

worin $R_3'$ $C_3$-$C_5$-Alkenyl bedeutet, in Gegenwart eines Katalysators hyriert, wobei $R_1$, $R_2$, $R_3$, $R_4$ und Y den unter Formel I angegebenen Bedeutungen entsprechen, W für -OH, -OM oder eine sogenannte Abgangsgruppe steht und Z die Gruppe -OM oder eine Abgangsgruppe darstellt, wobei M ein Alkali- oder Erdalkalimetallatom repräsentiert, mit der Massgabe, dass bei den Reaktanden Ia und III einerseits und V andererseits eine -OM-Gruppe mit einer Abgangsgruppe oder zwei Hydroxy-Gruppen miteinander reagieren.

12. Mittel zur Bekämpfung oder Verhütung eines Befalls durch Mikroorganismen, dadurch gekennzeichnet, dass es als aktive Komponente mindestens eine Verbindung der Formel I gemäss Anspruch 1 enthält.

13. Mittel gemäss Anspruch 12, dadurch gekennzeichnet, dass es als aktive Komponente mindestens eine Verbindung der Formel I' gemäss Anspruch 2 enthält.

14. Mittel nach Anspruch 12, dadurch gekennzeichnet, dass es 0,1 bis 99 % eines Wirkstoffes der Formel I, 99,9 bis 1 % eines festen oder flüssigen Zusatzstoffes und 0 bis 25 % eines Tensids enthält.

15. Mittel nach Anspruch 13, dadurch gekennzeichnet, dass es 0,1 bis 99 % eines Wirkstoffes der Formel I', 99,9 bis 1 % eines festen oder flüssigen Zusatzstoffes und 0 bis 25 % eines Tensids enthält.

16. Mittel nach Anspruch 14, dadurch gekennzeichnet, dass es 0,1 bis 95 % eines Wirkstoffes der Formel I, 99,8 bis 5 % eines festen oder flüssigen Zusatzstoffes und 0,1 bis 25 % eines Tensids enthält.

17. Mittel nach Anspruch 15, dadurch gekennzeichnet, dass es 0,1 bis 95 % eines Wirkstoffes der Formel I', 99,8 bis 5 % eines festen oder flüssigen Zusatzstoffes und 0,1 bis 25 % eines Tensids enthält.

18. Verfahren zur Bekämpfung oder Verhütung eines Befalls von Kulturpflanzen durch phytopathogene Mikroorganismen, dadurch gekennzeichnet, dass man eine gemäss Anspruch 1 definierte Verbindung der Formel I auf die Pflanze oder deren Standort appliziert.

19. Verfahren zur Bekämpfung oder Verhütung eines Befalls von Kulturpflanzen durch phytopathogene Mikroorganismen, dadurch gekennzeichnet, dass man eine gemäss Anspruch 2 definierte Verbindung der Formel I' auf die Pflanze oder deren Standort appliziert.

20. Verwendung von Verbindungen der Formel I gemäss Anspruch 1 zur Bekämpfung und/oder präventiven Verhütung eines Befalls von Mikroorganismen.

21. Verwendung von Verbindungen der Formel I' gemäss Anspruch 2 zur Bekämpfung und/oder präventiven Verhütung eines Befalls von Mikroorganismen.

Patentansprüche für folgende Vertragsstaaten: AT, ES

1. Mikrobizides Mittel enthaltend neben üblichen Träger- und Hilfsstoffen als Aktivkomponente mindestens eine Verbindung der Formel I

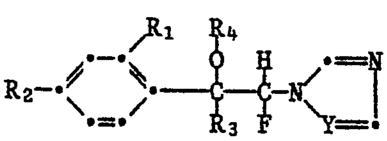

(I),

worin

$R_1$ Wasserstoff, Halogen, $C_1$-$C_2$-Alkyl oder $CF_3$;
$R_2$ Halogen oder $C_1$-$C_2$-Alkyl;
$R_3$ $C_1$-$C_5$-Alkyl oder $C_3$-$C_5$-Alkenyl;
$R_4$ Wasserstoff, $C_1$-$C_4$-Alkyl, $C_3$-$C_4$-Alkenyl oder die Gruppe

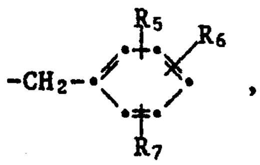

wobei
$R_5$, $R_6$ und $R_7$ unabhängig voneinander Wasserstoff, Nitro, Halogen, Cyano, $C_1$-$C_3$-Alkyl, $C_1$-$C_3$-Alkoxy, $C_1$-$C_3$-Haloalkoxy oder $CF_3$ darstellt, und
Y ein Stickstoffatom oder die CH-Gruppe bedeuten,
unter Einschluss ihrer Säureadditionssalze, quaternären Azoliumsalze und Metallsalzkomplexe.

2. Mittel gemäss Anspruch 1 enthaltend als Aktivkomponente mindestens eine Verbindung der Formel I′

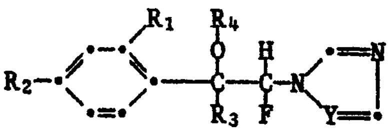

(I′),

worin
$R_1$ Wasserstoff, Halogen, $C_1$-$C_2$-Alkyl oder $CF_3$;
$R_2$ Halogen oder $C_1$-$C_2$-Alkyl;
$R_3$ $C_1$-$C_4$-Alkyl;
$R_4$ Wasserstoff, $C_1$-$C_4$-Alkyl, $C_3$-$C_4$-Alkenyl oder die Gruppe

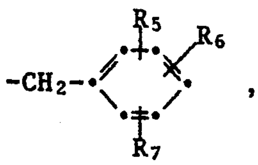

wobei
$R_5$, $R_6$ und $R_7$ unabhängig voneinander Wasserstoff, Nitro, Halogen, Cyano, $C_1$-$C_3$-Alkyl, $C_1$-$C_3$-Alkoxy, $C_1$-$C_3$-Haloalkoxy oder $CF_3$ darstellt, und
Y ein Stickstoffatom oder die CH-Gruppe bedeuten,
unter Einschluss ihrer Säureadditionssalze, quaternären Azoliumsalze und Metallsalzkomplexe.

3. Mittel gemäss Anspruch 2 enthaltend als Aktivkomponente mindestens eine Verbindung der Formel I′, worin
$R_1/R_2$ 2,4-$Cl_2$ oder 2,4-$(CH_3)_2$;
$R_3$ $C_1$-$C_2$-Alkyl; und
$R_4$ Wasserstoff, $C_1$-$C_3$-Alkyl, Allyl oder 2.4-Dichlorbenzyl
und
Y ein Stickstoffatom oder die CH-Gruppe
bedeuten.

4. Mittel gemäss Anspruch 2 enthaltend als Aktivkomponente mindestens eine Verbindung der Formel I′, worin
$R_1/R_2$ 2,4-$Cl_2$;
$R_3$ $CH_3$; und
$R_4$ Wasserstoff; und
Y ein Stickstoffatom
bedeuten.

5. Mittel gemäss Anspruch 1 enthaltend als Aktivkomponente mindestens eine Verbindung der Formel I′, worin
$R_1/R_2$ 2.4-$Cl_2$ oder 2,4-$(CH_3)_2$:
$R_3$ $C_1$-$C_5$-Alkyl oder $C_3$-$C_4$-Alkenyl:
$R_4$ Wasserstoff, $C_1$-$C_3$-Alkyl, Allyl oder 2,4-Dichlorbenzyl: und
Y ein Stickstoffatom oder die CH-Gruppe
bedeuten.

6. Mittel gemäss Anspruch 1 enthaltend als Aktivkomponente mindestens eine Verbindung der Formel I′. worin
$R_1$ Wasserstoff, Chlor oder Methyl;

$R_2$ Fluor, Chlor oder Methyl:
$R_3$ $C_1$-$C_4$-Alkyl oder Allyl;
$R_4$ $C_1$-$C_4$-Alkyl oder $C_3$-$C_4$-Alkenyl: und
Y ein Stickstoff oder die CH-Gruppe
bedeuten.

7. Mittel gemäss Anspruch 1 enthaltend als Aktivkomponente mindestens eine Verbindung der Formel I nach Anspruch 5, worin
$R_1/R_2$ 2.4-$Cl_2$:
$R_3$ $CH_3$: n-Propyl oder Allyl:
$R_4$ Wasserstoff oder Methyl: und
Y ein Stickstoffatom
bedeuten.

8. Mittel gemäss Anspruch 1 enthaltend als Aktivkomponente mindestens eine Verbindung der Formel I nach Anspruch 6. worin
$R_1$ Wasserstoff, Chlor oder Methyl;
$R_2$ Fluor, Chlor oder Methyl;
$R_3$ Methyl, n-Propyl, n-Butyl, tert. Butyl oder Allyl;
$R_4$ Methyl, n-Propyl, n-Butyl, Allyl oder But-2-enyl; und
Y ein Stickstoffatom oder die CH-Gruppe
bedeuten.

9. Mittel gemäss Anspruch 1 enthaltend als Aktivkomponente mindestens eine Verbindung der Formel I′ nach Anspruch 2 aus der Gruppe:
1-(2,4-Dichlorphenyl)-1-methyl-2-fluor-2-(1H-1,2,4-triazol-1-yl)-ethanol;
1-(2,4-Dichlorphenyl)-1-methyl-1-methoxy-2-fluor-2-(1H-1,2,4-triazol-1-yl)-ethan.

10. Mittel gemäss Anspruch 1 enthaltend als Aktivkomponente mindestens eine Verbindung der Formel I nach Anspruch 1 aus der Gruppe:
1-(2,4-Dichlorphenyl)-1-n-propyl-2-fluor-2-(1H-1,2,4-triazol-1-yl)-ethanol;
1-(1,2-Dichlorphenyl)-1-n-propyl)-2-fluor-2-(1H-imidazol-1-yl)-ethanol;
1-(2,4-Dimethylphenyl)-1-methyl-2-fluor-2-(1H-1,2,4-triazol-1-yl)-ethanol;
1-(2,4-Dimethylphenyl)-1-n-propyl-2-fluor-2-(1H-1,2,4-triazol-1-yl)-ethanol;
1-(4-fluorphenyl-1-methyl-2-fluor-2-(1H-1,2,4-triazol-1-yl)-ethanol;
1-(4-Chlorphenyl)-1-methyl-2-fluor-2-(1H-1,2,4-triazol-1-yl-ethanol;
1-(2-Chlor-4-fluorphenyl)-1-n-propyl-2-fluor-2-(1H-1,2,4-triazol-1-yl)-ethanol;
1-(2,4-Dichlorphenyl)-1-n-butyl-2-fluor-2-(1H-1,2,4-triazol-1-yl)-ethanol;
I-(4-Chlorphenyl)-1-t.butyl-2-fluor-2-(1H-1,2,4-triazol-1-yl)-ethanol;
1-(2,4-Dichlorphenyl)-1-allyl-2-fluor-2-(1H-1,2,4-triazol-1-yl)-ethanol;
1-(4-Chlorphenyl)-1-allyl-2-fluor-2-(1H-1,2,4-triazol-1-yl)-ethanol;
1-(2,4-Dichlorphenyl)-1-methyl-1-methoxy-2-fluor-2-(1H-1,2,4-triazol-1-yl)-ethan;
1-(2,4-Dichlorphenyl)-1-methyl-1-n-propoxy-2-fluor-2-(1H-1,2,4-triazol-1-yl)-ethan;
1-(2,4-Dichlorphenyl)-1-methyl-1-n-propoxy-2-(1H-imidazol-1-yl)-ethan;
1-(2,4-Dichlorphenyl)-1-methyl-1-n-butoxy-2-fluor-2-(1H-1,2,4-triazol-1-yl)-ethan;
1-(2,4-Dichlorphenyl)-1-methyl-1-allyloxy-2-fluor-2-(1H-1,2,4-triazol-1-yl)-ethan;
1-(2,4-Dichlorphenyl)-1-methyl-1-allyloxy-2-(1H-imidazol-1-yl)-ethan;
1-(2,4-Dichlorphenyl)-1-methyl-1-but-2-enoxy-2-fluor-2-(1H-1,2,4-triazol-1-yl)-ethan;
1-(2,4-Dichlorphenyl)-1-n-propyl-1-methoxy-2-fluor-2-(1H-1,2,4-triazol-1-yl)-ethan;
1-(2,4-Dichlorphenyl)-1-n-propyl-1-methoxy-2-(1H-imidazol-1-yl)-ethan;
1-(2,4-Dichlorphenyl)-1-n-propyl-1-n-propoxy-2-fluor-2-(1H-1,2,4-triazol-1-yl)-ethan;
1-(2,4-Dichlorphenyl)-1-n-propyl-1-allyloxy-2-fluor-2-(1H-1,2,4-triazol-1-yl)-ethan;
1-(2,4-Dimethylphenyl)-1-methyl-1-methoxy-2-fluor-2-(1H-1,2,4-triazol-1-yl)-ethan;
1-(2,4-Dichlorphenyl)-1-allyl-1-methoxy-2-fluor-2-(1H-1,2,4-triazol-1-yl)-ethan;
1-(2,4-Dichlorphenyl)-1-n-propyl-1-methoxy-2-fluor-2-(1H-1,2,4-triazol-1-yl)-ethan.

11. Verfahren zur Herstellung von Verbindungen der Formel I in Anspruch 1, dadurch gekennzeichnet, dass

a) ein Alkanolderivat der Formel Ia

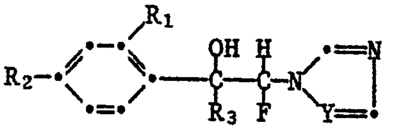

(Ia),

synthetisiert wird, indem man eine Ketonverbindung der Formel II

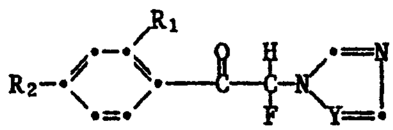 (II),

mit einer Verbindung der Formel IV
$R_3MgX$ (IV)
als Grignard-Reagens in einem inerten Lösungsmittel umsetzt, und

b) ein Etherderivat der Formel Ib

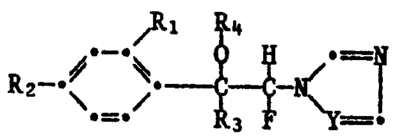 (Ib),

synthetisiert wird, indem man eine Alkanolverbindung der Formel Ia

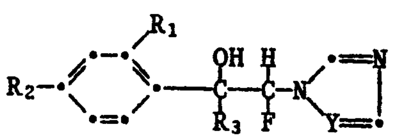 (Ia),

oder eine Verbindung der Formel III

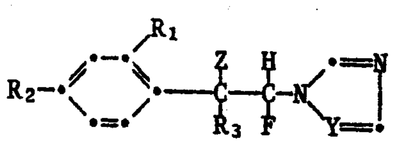 (III),

mit jeweils einer Verbindung der Formel V
$R_4-W$ (V)
reagieren lässt,
und

c) eine Verbindung der Formel Ic

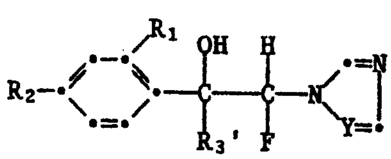 (Ic)

worin $R_3'$ $C_3$-$C_5$-Alkenyl bedeutet, synthetisiert wird, indem man eine Verbindung der Formel II

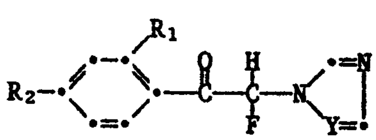 (II)

mit einer Silanverbindung der Formel VI $R_3'$-Si(Alk)$_3$ (VI)
worin Alk $C_1$-$C_4$-Alkyl, vorzugsweise Methyl, bedeutet, in Gegenwart einer Lewis-Säure umsetzt, und

d) eine Verbindung der Formel Id

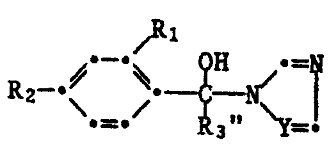 (Id)

worin $R_3''$ $C_3$-$C_5$-Alkyl bedeutet, hergestellt wird, indem man eine Verbindung der Formel Ic

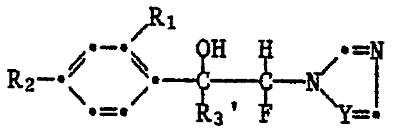

(Ic)

worin $R_3'$ $C_3$-$C_5$-Alkenyl bedeutet, in Gegenwart eines Katalysators hydriert, wobei $R_1$, $R_2$, $R_3$, $R_4$ und Y den unter Formel I angegebenen Bedeutungen entsprechen, W für -OH, -OM oder eine sogenannte Abgangsgruppe steht und Z die Gruppe -OM oder eine Abgangsgruppe darstellt, wobei M ein Alkali- oder Erdalkalimetallatom repräsentiert, mit der Massgabe, dass bei den Reaktanden Ia und III einerseits und V andererseits eine -OM-Gruppe mit einer Abgangsgruppe oder zwei Hydroxy-Gruppen miteinander reagieren.

12. Verfahren zur Bekämpfung oder Verhütung eines Befalls von Kulturpflanzen durch phytopathogene Mikroorganismen, dadurch gekennzeichnet, dass man eine gemäss Anspruch 1 definierte Verbindung der Formel I auf die Pflanze oder deren Standort appliziert.

13. Verfahren zur Bekämpfung oder Verhütung eines Befalls von Kulturpflanzen durch phytopathogene Mikroorganismen, dadurch gekennzeichnet, dass man eine gemäss Anspruch 2 definierte Verbindung der Formel I' auf die Pflanze oder deren Standort appliziert.

14. Verwendung von in Anspruch 1 definierten Verbindungen der Formel I zur Bekämpfung und/oder präventiven Verhütung eines Befalls von Mikroorganismen.

15. Verwendung von in Anspruch 2 definierten Verbindungen der Formel I, zur Bekämpfung und/oder präventiven Verhütung eines Befalls von Mikroorganismen.

Europäisches Patentamt

# EUROPÄISCHER RECHERCHENBERICHT

Nummer der Anmeldung

EP 86810588.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
|---|---|---|---|
| X | EP - A1 - 0 149 426 (CIBA)<br>* Ansprüche 1,10,11; Tabellen 2,6 *<br>-- | 1,12-21 | C 07 D 249/08<br>C 07 D 233/50<br>A 01 N 43/653<br>A 01 N 43/50 |
| A | EP - A2/A3 - 0 163 606 (CIBA)<br>* Formel II; Ansprüche 1,6 *<br>-- | 1,12 | |
| A | EP - A2/A3 - 0 153 803 (PFIZER)<br>* Anspruch 1 *<br>-- | 1 | |
| A | EP - A1 - 0 118 245 (PFIZER)<br>* Formel I; Tabelle Seite 31 *<br>---- | 1 | |

RECHERCHIERTE SACHGEBIETE (Int. Cl. 4)

C 07 D 249/00
C 07 D 233/00

Der vorliegende Recherchenbericht wurde für alle Patentansprüche erstellt.

| Recherchenort | Abschlußdatum der Recherche | Prüfer |
|---|---|---|
| WIEN | 03-03-1987 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument